(19) Europäisches Patentamt
European Patent Office
Office européen des brevets

(11) Veröffentlichungsnummer: **0 227 045 B1**

# (12) EUROPÄISCHE PATENTSCHRIFT

(45) Veröffentlichungstag der Patentschrift: **11.08.93**

(51) Int. Cl.[5]: **C07D 213/64**, A01N 43/40

(21) Anmeldenummer: **86117655.0**

(22) Anmeldetag: **18.12.86**

Die Akte enthält technische Angaben, die nach dem Eingang der Anmeldung eingereicht wurden und die nicht in dieser Patentschrift enthalten sind.

## (54) Heterocyclische Phenyläther, Verfahren zu deren Herstellung und diese enthaltende herbizide Mittel.

(30) Priorität: **21.12.85 DE 3545571**

(43) Veröffentlichungstag der Anmeldung:
**01.07.87 Patentblatt 87/27**

(45) Bekanntmachung des Hinweises auf die Patenterteilung:
**11.08.93 Patentblatt 93/32**

(84) Benannte Vertragsstaaten:
**AT BE CH DE ES FR GB GR IT LI NL**

(56) Entgegenhaltungen:
**EP-A- 0 109 483**
**FR-A- 2 412 532**

(73) Patentinhaber: **HOECHST AKTIENGESELLSCHAFT**

**W-6230 Frankfurt am Main 80(DE)**

(72) Erfinder: **Koch, Volker, Dr.**
**Altkönigstrasse 5**
**W-6233 Kelkheim(DE)**
Erfinder: **Willms, Lothar, Dr.**
**Schulstrasse 3**
**W-5411 Hillscheid(DE)**
Erfinder: **Fuss, Andreas, Dr.**
**Lindigstrasse 24**
**W-8757 Karlstein(DE)**
Erfinder: **Bauer, Klaus, Dr.**
**Kolpingstrasse 7**
**W-6054 Rodgau(DE)**
Erfinder: **Bieringer, Hermann, Dr.**
**Eichenweg 26**
**W-6239 Eppstein/Taunus(DE)**
Erfinder: **Bürstell, Helmut, Dr.**
**Am Hohlacker 65**
**W-6000 Frankfurt am Main(DE)**

EP 0 227 045 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft das Gebiet der Pflanzenschutzmittel, speziell der herbiziden heterocyclischen Phenyläthercarbonsäurederivate.

Aus FR-A-2412532 sind 4-(Pyrid-2-yloxy)-phenoxypropionsäurederivate bekannt, die in 5-Stellung am Pyridylring mit einer Trifluormethylgruppe substituiert sind und herbizide oder pflanzenwachstumsregulatorische Wirkungen aufweisen.

EP-A-0109483 beschreibt unter anderem herbizide [4-(Pyrid-2-yloxy)-phenoxy]-alkandione, die in 3,5-Position am Pyridylrest durch Reste aus der Gruppe Wasserstoff, Alkyl, Haloalkyl, Alkoxy, Haloalkoxy, Halogen, Nito und Cyano substituiert sind.

Gegenstand der vorliegenden Erfindung sind neue heterocyclisch substituierte 4-Phenoxyalkancarbonsäure derivate der allgemeinen Formel

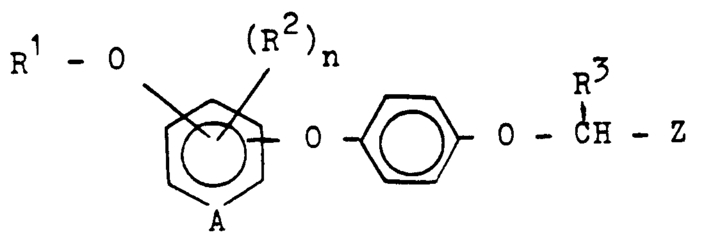

I

worin

| | |
|---|---|
| $R^1$ | Halogen($C_1$-$C_8$)alkyl, |
| $R^2$ | Halogen, |
| n | 0 bis 3, |
| A | N, |
| $R^3$ | $CH_3$, |
| Z | eine Gruppe der Formel |

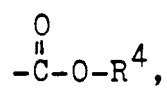, 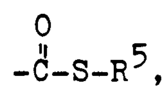, 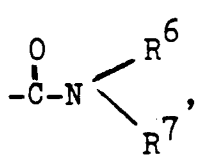, 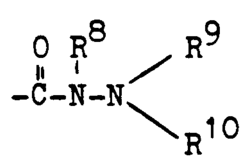,

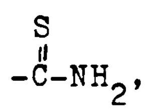, $-CN$

oder

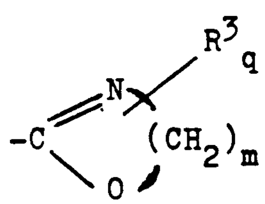,

| | |
|---|---|
| q | 0 bis 3, m = 2 oder 3, |
| $R^4$ | H, ($C_1$-$C_{12}$)-Alkyl, das gegebenenfalls durch 1 - 6 Halogen, vorzugsweise F, Cl, Br und/oder durch OH, CN, SCN, ($C_1$-$C_6$)-Alkoxy, ($C_1$-$C_4$)-Alkylthio, ($C_1$-$C_4$)-Alkylsulfinyl, ($C_1$-$C_4$)-Alkylsulfonyl, ($C_1$-$C_6$)-Alkoxy-($C_2$-$C_6$)-alkoxy, Halogen-($C_1$-$C_2$)-alkoxy, Methoxy-äthoxy- |

äthoxy, $(C_1-C_4)$-Alkylamino, Di-$(C_1-C_4)$-alkylamino, Phenyl, Oxiranyl, $(C_1-C_4)$-Alkoxycarbonyl und/oder den Rest

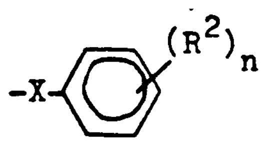

substituiert sein kann;
$(C_5-C_6)$-Cycloalkyl, das gegebenenfalls durch Halogen oder Methyl substituiert ist;
$(C_3-C_6)$-Alkenyl, Halogen-$(C_3-C_6)$-alkenyl oder $(C_5-C_6)$-Cycloalkenyl;
$(C_3-C_4)$-Alkinyl, das gegebenenfalls ein- oder zweifach durch $(C_1-C_6)$-Alkyl, Phenyl, Halogen bzw. $(C_1-C_2)$-Alkoxy substituiert ist;
Phenyl, das gegebenenfalls ein- bis dreifach durch $(C_1-C_4)$-Alkyl, $(C_1-C_4)$-Alkoxy, Halogen, $NO_2$ oder $CF_3$ substituiert ist;
Furfuryl, Tetrahydrofurfuryl, ein Kationäquivalent einer organischen oder anorganischen Base oder eine der Gruppen

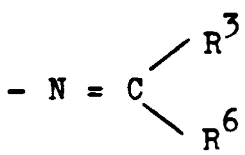

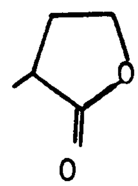

,

| | |
|---|---|
| $R^5$ | H, $(C_1-C_6)$-Alkyl, Phenyl-$(C_1-C_2)$-alkyl, wobei der Phenylrest ein- oder zweifach durch $(C_1-C_4)$-Alkyl und/oder Halogen substituiert sein kann; $(C_3-C_6)$-Alkenyl oder Phenyl, das auch ein- oder mehrfach durch $(C_1-C_4)$-Alkyl und/oder Halogen substituiert sein kann, |
| $R^6$ | H, $(C_1-C_6)$-Alkyl, Hydroxy-$(C_1-C_6)$-alkyl, $(C_5-C_6)$-Cycloalkyl oder Phenyl, das gegebenenfalls ein- bis dreifach durch $(C_1-C_4)$Alkyl, $(C_1-C_4)$-Alkoxy, Halogen oder $CF_3$ substituiert ist, |
| $R^7$ | eine der Bedeutungen von $R^6$ hat oder (wenn $R^6$ = H oder $(C_1-C_6)$-Alkyl) auch $(C_1-C_4)$-Alkoxycarbonyl-$(C_1-C_4)$-alkyl sein kann, |
| $R^6$ und $R^7$ | gemeinsam eine Alkylenkette mit 2, 4 oder 5 Gliedern, in der eine $CH_2$-Gruppe durch O, NH oder $N(CH_3)$ ersetzt sein kann, |
| $R^8$ | H oder $CH_3$, |
| $R^9$ | H, $CH_3$ oder $C_2H_5$, |
| $R^{10}$ | H, $CH_3$, $C_2H_5$ oder Phenyl |
| | sowie |
| X | O, S, N-$(C_1-C_4)$-Alkyl, NH, $-OCH_2-$, $-NHCH_2$, -N-($(C_1-C_4)$-Alkyl)-$CH_2$- oder $-SCH_2-$ bedeuten. |

Die Halogenalkyl-, -alkenyl- und -alkinylreste können sowohl geradkettig als auch verzweigt sein. Der Rest $R_1$ kann z.B. $CF_2H$, $CF_3$, $CF_2CHF_2$, $CF_2CHFCl$, $CF_2CHFBr$, $CF_2CHFCF_3$, $CH_2CF_3$, $CFCl_2$, $CF_2Cl$, $CH_2Cl$, $CHCl_2$, $CCl_3$, $CHBr_2$, $CH_2CCl=CClH$, $CH(CH_3)CH_2Cl$, $CH_2CHClCH_3$, $CH_2CH_2Cl$, $CH_2(CH_2)_2CH_2Cl$ bedeuten. Bevorzugt sind Alkylreste mit 1 - 2 C-Atomen und unter diesen wiederum Fluor- und/oder Chlorfluoralkyl.

Z ist bevorzugt ein Rest der Formel $COOR^4$, $COSR^5$ oder $CONR^6R^7$, wobei $COOR^4$ besonders bevorzugt ist. Als Rest $R^4$ kommt vor allem H, $(C_1-C_6)$-Alkyl, insbesondere Methyl oder Ethyl, Alkenyl (Allyl), Alkinyl (Propinyl), Halogenalkyl oder ein Kation in Frage.

Als Kationen seien die folgenden genannt: $Na^+$, $K^+$, $Ca^{++}$, $NH_4^+$, $^+NH_3CH_3$, $^+NH_2(CH_3)_2$, $^+NH(CH_3)_3$, $^+N(CH_3)_3$, $^+N(CH_3)_4$, $^+NH(C_2H_5)_3$, $^+N(C_2H_5)_4$, $^+NH(C_2H_4OH)_3$, $NH_2(C_6H_5)_2$, $NH_3CH_2C_6H_5$.

Gegenstand der Erfindung sind ferner Verfahren zur Herstellung der Verbindungen der Formel I, dadurch gekennzeichnet, daß man

a) Verbindungen der Formel

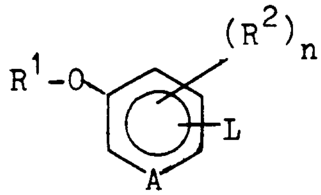

worin L eine Abgangsgruppe wie z.B. Halogen, $(C_1-C_4)$-Alkylsulfonyl, Phenylsulfonyl, $(C_1-C_4)$-Alkoxycarbonylmethylsulfonyl, $(C_1-C_4)$-Alkylsulfinyl, $(C_1-C_4)$-Alkylthio sowie den Mesyl- oder Tosylrest bedeutet, mit Verbindungen der Formel

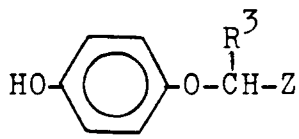 III

oder
b) Verbindungen der Formel

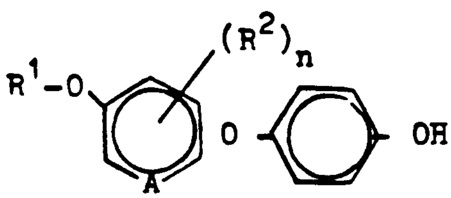 IV

mit Verbindungen der Formel

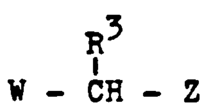 V

worin W für Halogen (vorzugsweise Chlor oder Brom) oder den Mesyl- bzw. Tosylrest steht, umsetzt, oder
c) die nach Verfahren a) und b) erhaltenen Verbindungen durch Verseifung, Salzbildung, Säurechloridherstellung und Umsetzung mit Alkoholen, Thiolen und Aminen, Veresterung, Umesterung, Amidierung, Wasserabspaltung, oder -anlagerung oder Schwefelwasserstoffanlagerung in andere erfindungsgemäße Verbindungen der Formel I überführt.

Zu a) und b)

Die Umsetzung der Verbindungen II und III sowie IV und V erfolgt vorzugsweise in inerten aprotischen Lösungsmitteln wie aliphatischen oder aromatischen Kohlenwasserstoffen (wie z.B. Benzol, Toluol, Xylol), Säurenitrilen wie z.B. Acetonitril oder Propionitril, Ketonen wie z.B. Aceton, Methyläthylketon oder Methylisobutylketon, Säureamiden wie z.B. Dimethylformamid, Dimethglacetamid, N-Methylpyrrolidon oder Hexamethglphosphorsäuretriamid oder in Dimethylsulfoxid bei Temperaturen zwischen 30° und 250°C bzw. der Siedetemperatur des Lösungsmittels, vorzugsweise zwischen 60° und 160°C, in Gegenwart anorganischer oder organischer Basen wie z.B. Metallalkoholaten, tertiären Aminen, Alkali- oder Erdalkalicarbonaten und -hydroxyden (NaOH, KOH).

Zu c)

Zur Amidierung von Verbindungen der Formel I kann man entweder von Estern ausgehen udn diese mit Aminen, Ammoniak oder Hydrazinen umsetzen. Man verwendet dabei vorzugsweise die gleichen Lösungsmittel wie bei a) und arbeitet bei Temperaturen zwischen 40 °C und Rückflußtemperatur. Man kann aber zunächst Säuren der Formel I in bekannter Weise in Säurehalogenide überführen und diese anschließend mit Ammoniak, Aminen oder Hydrazinen umsetzen. Zur Bindung des freiwerdenden Halogenwasserstoffes ist ein mindestens einmolarer Überschuß der eingesetzten Base erforderlich. Durch Umsetzung des Säurechlorids mit Alkoholen oder Mercaptanen kann man andere Ester oder Thioester der Formel I erhalten.

Die Umesterung geschieht durch saure oder basische Katalyse. Man setzt den Alkohol, der in den Ester eingeführt werden soll, zweckmäßigerweise im Überschuß zu und destilliert den freiwerdenden, niedriger siedenden Alkohol laufend in dem Maße ab, in dem er bei der Umesterung gebildet wird.

Bei der Entwässerung von Amiden zu Nitrilen arbeitet man vorzugsweise in aromatischen Kohlenwasserstoffen bei Temperaturen von 50 °C bis zur Siedetemperatur. Die anschließende Anlagerung von $H_2S$ erfolgt zweckmäßigerweise im Autoklaven in Gegenwart katalytischer Mengen einer Base (vorzugsweise Äthanolamin) bei Temperaturen zwischen 50 ° und 150 °C.

Die für die Herstellung der erfindungsgemäßen Verbindungen der allgemeinen Formel I benötigten Heterocyclen der Formel II stehen gemäß der Definition von X für entsprechend substituierte 2-Halogen-, 2-Alkylsulfonyl-, 2-Phenylsulfonyl-, 2-Alkoxycarbonylmethylsulfonyl-, 2-Alkylsulfinyl-, 2-Alkylthio, 2-Mesyl- oder 2-Tosyl- pyridin- oder pyridin-N-oxide die entsprechend HOE 85/F z.B. durch Anlagerung von Halogenalkenen oder -alkinen an entsprechend substituierte Halogenhydroxypyridine hergestellt werden können.

Die entsprechenden Phenole der Formeln III und IV lassen sich z.B. durch Monoalkylierung von Hydrochinon (J. Org. Chem. 39, S. 214 (1974) bzw. Soc. 1965, 954 - 73) herstellen.

Für $R_3$ Wasserstoff weisen die Verbindungen der Formel I ein Asymmetriezentrum auf und liegen üblicherweise in racemischer Form vor. Die Racemate lassen sich nach üblichen Methoden in Enantiomere trennen. Ebenso ist es jedoch auch möglich, nach den genannten Verfahren optisch aktive Ausgangsstoffe einzusetzen, insbesondere eignet sich dies für die Umsetzungen gemäß a) oder b) wobei die Verbindungen III und V in optisch aktiver Form eingesetzt werden. Gegenstand der Erfindung sind sowohl die Racemate als auch die optischen Antipoden, besonders deren D-Formen.

Die erfindungsgemäßen Verbindungen der allgemeinen Formel I sind im Vor- und Nachauflaufverfahren gegen ein breites Spektrum von ein- und mehrjährigen Schadgräsern sehr gut wirksam, gleichzeitig werden sie jedoch von zweikeimblättrigen Kulturpflanzen sowie einigen Getreidearten vorzüglich toleriert. Die Verbindungen sind daher z.B. zur selektiven Anwendung in Kulturpflanzen gegen Wildhafer (Avena), Fuchsschwanz (alopecurus spp.), Rispengras (Poa ssp.), Raygras (Lolium spp.), ein- und mehrjährige Wildhirsen (Ehinochloa spp., Setaria spp., Digitaria spp., Napicum spp., Sorghum ssp.), Bermudagras (Cynon spp.) und Quecke (Agropyron spp.) geeignet.

Bei Anwendung der erfindungsgemäßen Verbindungen in subtoxischen Dosen lassen sich typische wachstumsregulierende Effekte feststellen; so können beispielsweise der Wuchs der Pflanzen, aber auch die Pflanzeninhaltsstoffe beeinflußt werden. Damit eignen sich die Verbindungen als Wachstumsregulatoren in Nutzpflanzenkulturen wie z.B. Getreide, Mais, Zuckerrohr, Tabak, Reis und Sorghum. Andererseits lassen sich auch Pflanzenbestände regulieren wie Kulturrasen oder auch Pflanzengemeinschaften an Wege- und Straßenrändern sowie Zierpflanzen.

Gegenstand der vorliegenden Erfindung sind daher auch herbizide und wuchsregulierende Mittel, die dadurch gekennzeichnet sind, daß sie eine herbizide oder wuchsregulierend wirksame Menge einer Verbindung der allgemeinen Formel I neben üblichen Zusatz- und Formulierungshilfsmitteln enthalten.

Die erfindungsgemäßen Mittel enthalten die Wirkstoffe der Formel I im allgemeinen zu 2 - 95 Gew.-%. Sie können als benetzbare Pulver, emulgierbare Konzentrate, versprühbare Lösungen, Stäubemittel oder Granulate in den üblichen Zubereitungen angewendet werden.

Benetzbare Pulver sind in Wasser gleichmäßig dispergierbare Präparate, die neben dem Wirkstoff außer einem Verdünnungs- oder Inertstoff noch Netzmittel, z.B. polyoxäthylierte Alkylphenole, polyoxäthylierte Oleyl-, Stearylamine, Alkyl- oder Alkylphenyl-sulfonate und Dispergiermittel, z.B. ligninsulfonsaures Natrium, 2,2'-dinapthylmethan-6,6'-disulfonsaures Natrium, oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten und Zusatz eines nichtionischen Netzmittels, beispielsweise eines polyoxäthylierten Alkylphenols oder eines polyoxäthylierten Oleyl- oder Stearylamins, erhalten.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Versprühbare Lösungen, wie sie vielfach in Sprühdosen gehandelt werden, enthalten den Wirkstoff in einem organischen Lösungsmittel gelöst, daneben befindet sich z.B. als Treibmittel ein Gemisch von Fluorchlorkohlenwasserstoffen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacylsaurem Natrium, oder auch Mineralölen auf die Oberfläche von Trägerstoffen, wie Sand, Kaolinite, oder von granuliertem Inertmaterial. Auch können geeignete Wirkstoffe in der für die Herstlelung von Düngemittelgranalien üblichen Weise - gewünschtenfalls in Mischung mit Düngemitteln - hergestellt werden.

Bei den herbiziden Mitteln können die Konzentrationen der Wirkstoffe in den handelsüblichen Formulierungen verschieden sein. In benetzbaren Pulvern variiert die Wirkstoffkonzentrationen z.B. zwischen 10 % und 95 %, der Rest besteht aus den oben angegebenen Formulierungszusätzen. Bei emulgierbaren Konzentraten ist die Wirkstoffkonzentration etwa 10 % bis 80 %. Staubförmige versprühbare Lösungen etwa 2 bis 20 %. Bei Granulaten hängt der Wirkstoffgehalt z.T. davon ab, ob die wirksame Verbindung flüssig oder fest vorliegt und welche Granulierhilfsmittel Füllstoffe usw. verwendet werden.

Zur Anwendung werden die handelsüblichen Konzentrate gegebenenfalls in üblicher Weise verdünnt, z.B. bei benetzbaren Pulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperaturen, Feuchtigkeit u.a. variiert die erforderliche Aufwandmenge. Sie kann innerhalb weiter Grenzen schwanken und liegt für herbizide Mittel bei 0,01 und 10 kg/ha, führ wachstumsregulierende Mittel bei 0,001 bis 1 kg/ha.

Die erfindungsgemäßen Wirkstoffe können mit anderen Herbiziden, Insektiziden und Fungiziden kombiniert werden.

## A. FORMULIERUNGSBEISPIELE

### Beispiel A

Ein emulgierbares Konzentrat wird erhalten aus
15 Gew.-Teilen Wirkstoff
75 Gew.-Teilen Cyclohexanon als Lösungsmittel
und 10 Gew.-Teilen oxäthyliertes Nonylphenol (10 AeO) als Emulgator.

### Beispiel B

Ein in Wasser leicht dispergierbares benetzbares Pulver wird erhalten, indem man
25 Gew.-Teile Wirkstoff
64 Gew.-Teile kaolinhaltigen Quarz als Inertstoff
10 Gew.-Teile ligninsulfonsaures Calcium
und 1 Gew.-Teil oleylmethyltaurinsaures Natrium als Netz- und Dispergiermittel
mischt und in einer Stiftmühle mahlt.

### Beispiel C

Ein Stäubemittel wird erhalten, indem man
10 Gew.-Teile Wirkstoff,
und 90 Gew.-Teile Talkum als Inertstoff
mischt und in einer Schlagmühle zerkleinert.

### Beispiel D

Ein Granulat besteht z.B. aus etwa
2 - 15 Gew.-Teilen Wirkstoff
98 - 85 Gew.-Teilen inerten Granulatmaterialien, wie z.B. Attapulgit, Bimsstein und Quarzsand.

## B. CHEMISCHE BEISPIELE

### Beispiel 1

2-(4-(5-Chlor-3-(2-chlor-1,1,2-trifluorethoxy)-2-pyridyloxy)-phenoxy)propionsäureethylester

9,75 g (30 mmol) 2-Brom-5-chlor-3-(2-chlor-1,1,2-trifluorethoxy)pyridin, 6,94 g (33 mmol) 4-Hydroxyphenoxypropionsäureethylester und 6,6 g wasserfreier Kaliumcarbonat in 40 ml absolutem Dimethylsulfoxid wurden 9 h bei 80 °C unter Argon gerührt. Die Mischung wurde nach dem Abkühlen mit 300 ml Diethylester verdünnt, das Kaliumcarbonat wurde abfiltriert, die organische Phase mit 5 %iger KOH und anschließend mit Wasser neutral gewaschen. Nach dem erhielt man die Verbindung von Beispiel 1 in einer Rohausbeute von 13,4 g (98 % d. Th.)

Das Rohprodukt wurde über Kieselgel chromatographiert (Eluens Methylenchlorid). Das gereinigte Produkt hat einen $n_D^{20}$ von 1,5227. Die Reinheit bzw. Struktur wurde - wie auch bei den folgenden Verbindungen - durch Dünnschichtchromatographie bzw. NMR-Spektroskopie gesichert.

In analoger Weise erhält man die folgenden Verbindungen:

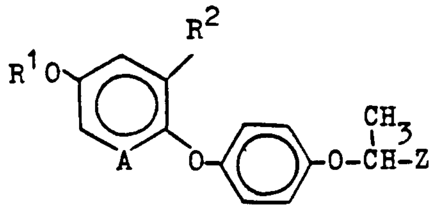

Tabelle 1

| lfd. Bsp. | $R^1$ | A | $R^2$ | Z | Kp; Fp; $n_D$ [°C] |
|---|---|---|---|---|---|
| 2 | $CF_2H$ | N | Cl | $COOC_2H_5$ | $n_D^{20}$ : 1.4898 |
| 3 | $CF_2CHClF$ | N | Cl | $COOC_2H_5$ | $n_D^{20}$ : 1.5000 |
| 4 | $CF_2CHF_2$ | N | Cl | $COOC_2H_5$ | $n_D^{20}$ : 1.5030 |
| 5 | $CF_2H$ | N | Cl | COOH | |
| 6 | $CF_2H$ | N | Cl | $COOCH_3$ | |
| 7 | $CF_2H$ | N | Cl | $COOC_3H_7$ | |
| 8 | $CF_2H$ | N | Cl | $COOC_4H_9$ | |
| 9 | $CF_2H$ | N | Cl | $COOCH_2CH_2OCH_3$ | |
| 10 | $CF_3$ (D-Isomeres) | N | Cl | $COOC_2H_5$ | $[\alpha]_D^{20}$ : 21,6° ($C = 20$; $CH_2Cl_2$) |
| 11 | $CF_3$ | N | H | $COOC_2H_5$ | $n_D^{20}$ : 1,5280 |
| 12 | $CF_2H$ | N | Cl | $COOCH(CH_3)C_2H_5$ | |
| 13 | $CF_2H$ | N | Cl | COO-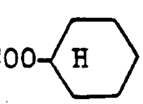 | |
| 14 | $CF_2H$ | N | Cl | $COOCH_2CH=CH_2$ | |

| Lfd. Bsp. | $R^1$ | A | $R^2$ | Z | Kp; Fp; $n_D$ [°C] |
|---|---|---|---|---|---|
| 15 | $CF_2H$ | N | Cl | $COON=C(CH_3)_2$ | |
| 16 | $CF_2H$ | N | Cl | $COSC_2H_5$ | |
| 17 | $CF_2H$ | N | Cl | $COOCH_2CH_2Cl$ | |
| 18 | $CF_2H$ | N | Cl | $COOCH_2-C_6H_5$ | |
| 19 | $CF_2H$ | N | Cl | $COOCH_2COOH_3$ | |
| 20 | $CF_2H$ | N | Cl | $COOCH(CH_3)COOC_2H_5$ | |
| 21 | $CF_2H$ | N | Cl | $CONH_2$ | |
| 22 | $CF_2H$ | N | Cl | $CONHCH_3$ | |
| 23 | $CF_2H$ | N | Cl | $CON(CH_3)_2$ | |
| 24 | $CF_2H$ | N | Cl | $CONHC_6H_5$ | |
| 24 | $CF_2H$ | N | Cl | CO 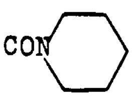 | |
| 26 | $CF_2H$ | N | H | $COOC_2H_5$ | |
| 27 | $CF_2H$ | N | H | $COOCH_3$ | |
| 28 | $CF_2H$ | N | H | COOH | |
| 29 | $CF_2H$ | N | Cl | COOK | |
| 30 | $CCl_3$ | N | Cl | $COOC_2H_5$ | |
| 31 | $CF_3$ | N | Cl | $COOC_3H_7$ (n) | |

| lfd. Bsp. | $R^1$ | A | $R^2$ | Z | Kp; Fp; $n_D$ [°C] |
|---|---|---|---|---|---|
| 32 | $CF_3$ | N | Cl | $COOC_2H_5$ | |
| 33 | $CF_2Cl$ | N | Cl | $COOCH_2CH{=}CH_2$ | |
| 34 | $CF_2H$ | N | Cl | $COOCH_2CH_2OCH_2C_6H_5$ | |
| 35 | $CF_2H$ | N | Cl | $COOCH_2CH_2N(CH_3)CH_2C_6H_5$ | |
| 36 | $CF_3$ | N | Cl | $COOCH_2SCH_3$ | |
| 37 | $CF_2CF_2H$ | N | Cl | COOH | |
| 38 | $CF_2Cl$ | N | Cl | $COOC_2H_5$ | $n_D^{20}$ : 1,5970 |
| 39 | $CF_2CHClF$ | N | Cl | $COOC_2H_5$ | |
| 40 | $CH_2CF_3$ | N | Cl | COONa | |
| 41 | $CH_2CH_2Cl$ | N | Cl | $COOC_2H_5$ | |
| 42 | $CH_2CH_2F$ | N | Cl | $COOH_3$ | |
| 43 | $CF_2H$ | N | Cl | $COOC_2H_5$ (D-Isomer) | |

## C. BIOLOGISCHE BEISPIELE

### Beispiel I Vorauflaufbehandlung

Samen von Gräsern wurden in Töpfen ausgesät und die als Spritzpulver bzw. als Emulsionskonzentrate formulierten erfindungsgemäßen Präparate in verschiedenen Dosierungen auf die Kulturboden-Oberfläche gesprüht. Anschließend wurden die Töpfe für 4 Wochen in einem Gewächshaus aufgestellt und das Resultat der Behandlung (ebenso wie bei den folgenden Beispielen) durch eine Bonitierung nach dem nachstehenden Schema festgehalten:

0 0 % Schädigung
1 > 0 - 20 % Schädigung
2 >20 - 40 % Schädigung
3 >40 - 60 % Schädigung
4 >60 - 80 % Schädigung
5 > 80 - 100 % Schädigung

Die erfindungsgemäßen Präparate zeigten eine gute Wirkung gegen einjährige und zum Teil auch gegen mehrjährige Schadgräser, als Testpflanzen wurden verwendet Avena, Alopecurus, Lolium, Echinochloa, Setaria, Agropyron und Cynodon.

Die erfindungsgemäßen Wirkstoffe zeigten in diesem Testmodell gute Wirkung (siehe Tabelle 2).

Beispiel II Nachauflaufbehandlung

Samen von Gräsern wurden in Töpfen ausgesät und im Gewächshaus angezogen. 3 Wochen nach der Aussaat wurden die als Spritzpulver bzw. als Emulsionskonzentrate formulierten erfindungsgemäßen Präparate in verschiedenen Dosierungen auf die Pflanzen gesprüht und nach 4 Wochen Standzeit im Gewächshaus die Wirkung der Präparate bonitiert.

Die erfindungsgemäßen Wirkstoffe waren gut gegen ein breites Spektrum von einjährigen Schadgräsern herbizid wirksam (siehe Tabelle 3). Einige Verbindungen bekämpften ferner auch die mehrjährigen Schadgräser Cynodon dactylon, Sorghum halepense sowie Agropyron repens.

Tabelle 2:

Vorauflaufwirkung

| Beispiel | Dosis kg a.i./ha | herbizide Wirkung POA | AVF | ALM | SAL | LOM | ECG |
|---|---|---|---|---|---|---|---|
| 2 | 2,4 | 5 | 5 | 5 | 5 | 5 | 5 |
| | 0,6 | 5 | 5 | 5 | 5 | 5 | 5 |
| 4 | 2,4 | 5 | 5 | 5 | 5 | 5 | 5 |
| 10 | 2,4 | 5 | 5 | 5 | 5 | 5 | 5 |
| | 0,6 | 5 | 5 | 5 | 5 | 5 | 5 |
| 38 | 2,4 | 5 | 5 | 5 | 5 | 5 | 5 |
| | 0,6 | 5 | 3 | 5 | 5 | 3 | 4 |

Tabelle 3:

Nachauflaufwirkung

| Beispiel | Dosis kg a.i./ha | herbizide Wirkung POA | AVF | ALM | SAL | LOM | ECG |
|---|---|---|---|---|---|---|---|
| 2 | 2,4 | 5 | 5 | 5 | 5 | 5 | 5 |
| | 0,6 | 5 | 5 | 5 | 5 | 5 | 5 |
| 4 | 2,4 | - | 5 | 5 | 5 | 5 | 5 |
| | 0,6 | - | 5 | 5 | 3 | 4 | 5 |
| 10 | 2,4 | 5 | 5 | 5 | 5 | 5 | 5 |
| | 0,6 | 5 | 5 | 5 | 5 | 5 | 5 |
| 38 | 2,4 | 5 | 5 | 5 | 5 | 5 | 5 |
| | 0,6 | 5 | 5 | 5 | 5 | 5 | 5 |

Abkürzungen:

POA = Poa annua (Rispengras)
AVF = Avena fatua (Flughafer)
ALM = Alopecurus myosuroides (Ackerfuchsschwanz)
SAL = Setaria lutescens (Bluthirse)
LOM = Lolium multiflorum (Raygras)
ECG = Echinochloa crus-galli (Hühnerhirse)
a.i.= Aktivsubstanz

Beispiel III Kulturpflanzenverträglichkeit

In weiteren Versuchen im Gewächshaus wurden Samen einer größeren Anzahl von Kulturpflanzen in Töpfen ausgelegt. Ein Teil der Töpfe wurde sofort behandelt, die übrigen wurden im Gewächshaus aufgestellt, bis die Pflanzen 2 bis 3 echte Blätter entwickelt hatten und dann mit erfindungsgemäßen Substanzen besprüht.

Die Ergebnisse, die 4 bis 5 Wochen nach Applikation festgestellt wurden, zeigen, daß die erfindungsgemäßen Substanzen zweikeimblättrige Kulturen im Vor- und Nachauflauf-Verfahren ungeschädigt lassen.

Einige Substanzen schonen darüber hinaus auch Gramineen-Kulturen wie Gerste, Sorghum, Mais, Weizen oder Reis.

Tabelle 4:

| Beispiel | Dosis kg/ha | Wirkung gegen BV | GS | BN |
|---|---|---|---|---|
| 2 | 2,4 | 0 | 0 | 0 |
| 4 | 2,4 | 0 | 0 | 0 |
| 10 | 2,4 | 0 | 0 | 0 |
| 38 | 2,4 | 0 | 0 | 0 |

Abkürzungen:
BV= Beta vulgaris (Zuckerrübe)
GS= Glycine soja (Soya)
BN= Brassica napa (Raps)

## Patentansprüche

### Patentansprüche für folgende Vertragsstaaten : BE, CH, DE, FR, GB, GR, IT, LI, NL

**1.** Verbindung der Formel I,

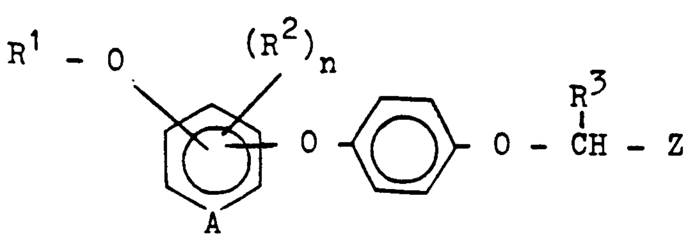

I

worin
R[1] Halogen($C_1$-$C_8$)alkyl,
R[2] Halogen,
n 0 bis 3,
A N,
R[3] $CH_3$,
Z eine Gruppe der Formel

-CO-O-$R^4$, -CO-S-$R^5$, -CO-N$R^6$$R^7$, -CO-N$R^8$-N$R^8$$R^{10}$, -CS-$NH_2$, CN

oder

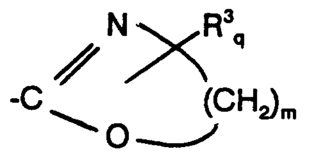 ,

q 0 bis 3,

m 2 oder 3,

$R^4$ H, $(C_1\text{-}C_{12})$-Alkyl, das gegebenenfalls durch 1 - 6 Halogen, vorzugsweise F, Cl, Br und/oder durch OH, CN, SCN, $(C_1\text{-}C_6)$-Alkoxy, $(C_1\text{-}C_4)$-Alkylthio, $(C_1\text{-}C_4)$-Alkylsulfinyl, $(C_1\text{-}C_4)$-Alkylsulfonyl, $(C_1\text{-}C_6)$-Alkoxy-$(C_2\text{-}C_6)$-alkoxy, Halogen-$(C_1\text{-}C_2)$-alkoxy, Methoxy-äthoxy-äthoxy, $(C_1\text{-}C_4)$-Alkylamino, Di-$(C_1\text{-}C_4)$-alkylamino, Phenyl, Oxiranyl, $(C_1\text{-}C_4)$-Alkoxycarbonyl und/oder den Rest

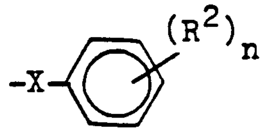

substituiert sein kann;
$(C_5\text{-}C_6)$-Cycloalkyl, das gegebenenfalls durch Halogen oder Methyl substituiert ist;
$(C_3\text{-}C_6)$-Alkenyl, Halogen-$(C_3\text{-}C_6)$-alkenyl oder $(C_5\text{-}C_6)$-Cycloalkenyl;
$(C_3\text{-}C_4)$-Alkinyl, das gegebenenfalls ein- oder zweifach durch $(C_1\text{-}C_6)$-Alkyl, Phenyl, Halogen bzw. $(C_1\text{-}C_2)$-Alkoxy substituiert ist;
Phenyl, das gegebenenfalls ein- bis dreifach durch $(C_1\text{-}C_4)$-Alkyl, $(C_1\text{-}C_4)$-Alkoxy, Halogen, $NO_2$ oder $CF_3$ substituiert ist;
Furfuryl, Tetrahydrofurfuryl, ein Kationäquivalent einer organischen oder anorganischen Base oder eine der Gruppen

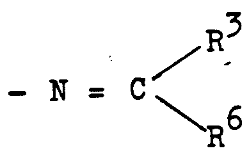 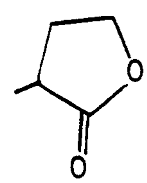 ,

$R^5$ H, $(C_1\text{-}C_6)$-Alkyl, Phenyl-$(C_1\text{-}C_2)$-alkyl, wobei der Phenylrest ein- oder zweifach durch $(C_1\text{-}C_4)$-Alkyl und/oder Halogen substituiert sein kann; $(C_3\text{-}C_6)$-Alkenyl oder Phenyl, das auch ein- oder mehrfach durch $(C_1\text{-}C_4)$-Alkyl und/oder Halogen substituiert sein kann,

$R^6$ H, $(C_1\text{-}C_6)$-Alkyl, Hydroxy-$(C_1\text{-}C_6)$-alkyl, $(C_5\text{-}C_6)$-Cycloalkyl oder Phenyl, das gegebenenfalls ein- bis dreifach durch $(C_1\text{-}C_4)$Alkyl, $(C_1\text{-}C_4)$-Alkoxy, Halogen oder $CF_3$ substituiert ist,

$R^7$ eine der Bedeutungen von $R^6$ hat oder (wenn $R^6$ = H oder $(C_1\text{-}C_6)$-Alkyl) auch $(C_1\text{-}C_4)$-Alkoxycarbonyl-$(C_1\text{-}C_4)$-alkyl sein kann,

$R^6$ und $R^7$ gemeinsam eine Alkylenkette mit 2, 4 oder 5 Gliedern, in der eine $CH_2$-Gruppe durch O, NH oder $N(CH_3)$ ersetzt sein kann,

$R^8$ H oder $CH_3$,

$R^9$ H, $CH_3$ oder $C_2H_5$,

$R^{10}$ H, $CH_3$, $C_2H_5$ oder Phenyl,
sowie

X O, S, N-$(C_1\text{-}C_4)$-Alkyl, NH, $-OCH_2-$, $-NHCH_2$, $-N((C_1\text{-}C_4)\text{-Alkyl})\text{-}CH_2-$ oder $-SCH_2-$ bedeuten.

**2.** Verbindungen der Formel I gemäß Anspruch 1, dadurch gekennzeichnet, daß $R^1$ ($C_1$-$C_2$) Halogenalkyl und $R^2$ Halogen, vorzugsweise Chlor ist.

**3.** Verbindungen der Formel I gemäß den Ansprüchen 1 oder 2, dadurch gekennzeichnet, daß Z ein Rest der Formel $-COOR^4$, $-COSR^5$ oder $-CONR^6R^7$, insbesondere aber $-COOR^4$ ist.

**4.** Verbindungen der Formel I gemäß einem der Ansprüche 1, 2 oder 3, dadurch gekennzeichnet, daß $R^4$ Wasserstoff, ($C_1$-$C_6$)-Halogenalkyl oder ein Kation ist.

**5.** Verfahren zur Herstellung der Verbindungen der Formel I, dadurch gekennzeichnet, daß man
a) Verbindungen der Formel

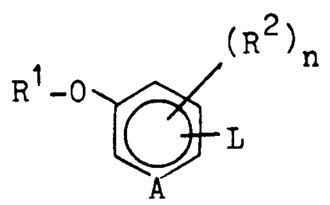

worin L eine Abgangsgruppe wie z.B. Halogen, ($C_1$-$C_4$)-Alkylsulfonyl, Phenylsulfonyl, ($C_1$-$C_4$)-Alkoxycarbonylmethylsulfonyl, ($C_1$-$C_4$)-Alkylsulfinyl, ($C_1$-$C_4$)-Alkylthio sowie den Mesyl- oder Tosylrest bedeutet, mit Verbindungen der Formel

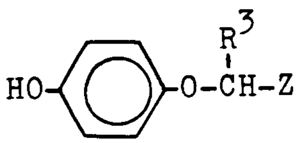

III

oder
b) Verbindungen der Formel

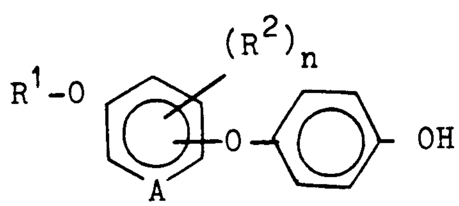

IV

mit Verbindungen der Formel

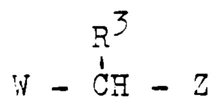

V

worin W für Halogen (vorzugsweise Chlor oder Brom) oder den Mesyl- bzw. Tosylrest steht, umsetzt, oder
c) Verbindungen der Formel I, in der z die $-COOR^4$-Gruppe darstellt, hydriert und die erhaltenen Alkohole ($Z = -CH_2OH$) gewünschtenfalls durch Umsetzung mit Carbonsäuren, Carbonsäurehalogeniden oder Anhydriden in die entsprechenden Carbonsäureester ($Z = -CH_2-O-C-(O)-R^{11}$), durch Umsetzung mit Sulfonsäurehalogeniden in Sulfonsäureester ($Z = -CH_2-O-SO_2-R^{12}$) bzw. durch Umsetzung mit Carbamidsäurehalogeniden oder Isocyanaten in Carbamidsäureester

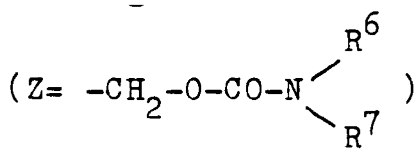

überführt, oder

d) nach Verfahren a) und b) erhaltenen Verbindungen durch Verseifung, Salzbildung, Säurechloridherstellung und Umsetzung mit Alkoholen, Thiolen und Aminen, Veresterung, Umesterung, Amidierung, Wasserabspaltung, oder -anlagerung oder Schwefelwasserstoffanlagerung in andere erfindungsgemäße Verbindungen der Formel I überführt.

**6.** Herbizide und wachstumsregulierende Mittel, gekennzeichnet durch einen Gehalt an einer Verbindung der Formel I gemäß Anspruch 1.

**7.** Verwendung von Verbindungen der Formel I gemäß Anspruch 1 als Herbizide bzw. als Wachstumsregulatoren.

**8.** Verfahren zur Bekämpfung unerwünschten Pflanzenwuchses, bzw. zur Wachstumsregulierung, dadurch gekennzeichnet, daß man auf die zu behandelnden Pflanzen bzw. Anbauflächen eine wirksame Menge einer Verbindung der Formel I gemäß Anspruch 1 aufbringt.

**Patentansprüche für folgende Vertragsstaaten : AT, ES**

**1.** Verfahren zur Herstellung von Verbindungen der allgemeinen Formel I

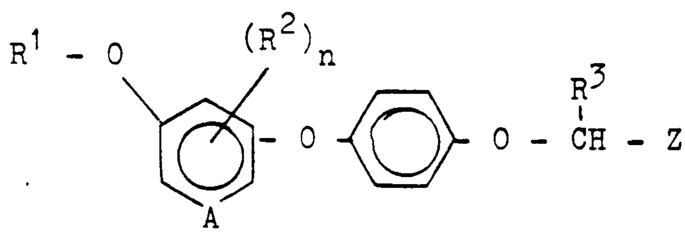

I

worin

| | |
|---|---|
| $R^1$ | Halogen($C_1$-$C_8$)alkyl, |
| $R^2$ | Halogen, |
| n | 0 bis 3, |
| A | N, |
| $R^3$ | $CH_3$ |
| Z | eine Gruppe der Formel |

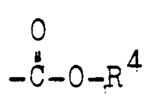, 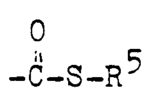, 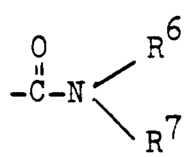, 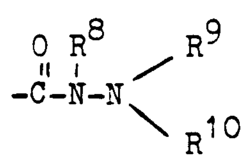,

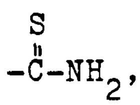, -CN,

oder

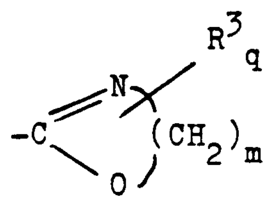,

q — 0 bis 3, m = 2 oder 3,

$R^4$ — H, ($C_1$-$C_{12}$)-Alkyl, das gegebenenfalls durch 1 - 6 Halogen, vorzugsweise F, Cl, Br und/oder durch OH, CN, SCN, ($C_1$-$C_6$)-Alkoxy, ($C_1$-$C_4$)-Alkylthio, ($C_1$-$C_4$)-Alkylsulfinyl, ($C_1$-$C_4$)-Alkylsulfonyl, ($C_1$-$C_6$)-Alkoxy-($C_2$-$C_6$)-alkoxy, Halogen-($C_1$-$C_2$)-alkoxy, Methoxyäthoxy-äthoxy, ($C_1$-$C_4$)-Alkylamino, Di-($C_1$-$C_4$)-alkylamino, Phenyl, Oxiranyl, ($C_1$-$C_4$)-Alkoxycarbonyl und/oder den Rest

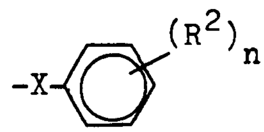

substituiert sein kann;
($C_5$-$C_6$)-Cycloalkyl, das gegebenenfalls durch Halogen oder Methyl substituiert ist;
($C_3$-$C_6$)-Alkenyl, Halogen-($C_3$-$C_6$)-alkenyl oder ($C_5$-$C_6$)-Cycloalkenyl;
($C_3$-$C_4$)-Alkinyl, das gegebenenfalls ein- oder zweifach durch ($C_1$-$C_6$)-Alkyl, Phenyl, Halogen bzw. ($C_1$-$C_2$)-Alkoxy substituiert ist;
Phenyl, das gegebenenfalls ein- bis dreifach durch ($C_1$-$C_4$)-Alkyl, ($C_1$-$C_4$)-Alkoxy, Halogen, $NO_2$ oder $CF_3$ substituiert ist;
Furfuryl, Tetrahydrofurfuryl, ein Kationäquivalent einer organischen oder anorganischen Base oder eine der Gruppen

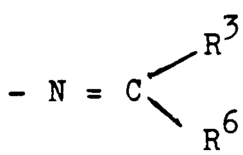

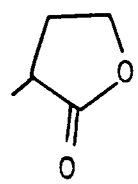,

$R^5$ — H, ($C_1$-$C_6$)-Alkyl, Phenyl-($C_1$-$C_2$)-alkyl, wobei der Phenylrest ein- oder zweifach durch ($C_1$-$C_4$)-Alkyl und/oder halogen substituiert sein kann; ($C_3$-$C_6$)-Alkenyl oder Phenyl, das auch ein- oder mehrfach durch ($C_1$-$C_4$)-Alkyl und/oder Halogen substituiert sein kann,

$R^6$ — H, ($C_1$-$C_6$)-Alkyl, Hydroxy-($C_1$-$C_6$)-alkyl, ($C_5$-$C_6$)-Cycloalkyl oder Phenyl, das gegebenenfalls ein- bis dreifach durch ($C_1$-$C_4$)Alkyl, ($C_1$-$C_4$)-Alkoxy, Halogen oder $CF_3$ substituiert ist,

$R^7$ — eine der Bedeutungen von $R^6$ hat oder (wenn $R^6$ = H oder ($C_1$-$C_6$)-Alkyl) auch ($C_1$-$C_4$)-Alkoxycarbonyl-($C_1$-$C_4$)-alkyl sein kann,

$R^6$ und $R^7$ — gemeinsam eine Alkylenkette mit 2, 4 oder 5 Gliedern, in der eine $CH_2$-Gruppe durch O, NH oder N($CH_3$) ersetzt sein kann,

$R^6$ — H oder $CH_3$,

$R^9$ — H, $CH_3$ oder $C_2H_5$,

$R^{10}$ — H, $CH_3$, $C_2H_5$ oder Phenyl,
sowie

X — O, S, N-($C_1$-$C_4$)-Alkyl, NH, -$OCH_2$-, -$NHCH_2$, -N-(($C_1$-$C_4$)-Alkyl)-$CH_2$- oder -$SCH_2$- bedeuten, dadurch gekennzeichnet, daß man

a) Verbindungen der Formel

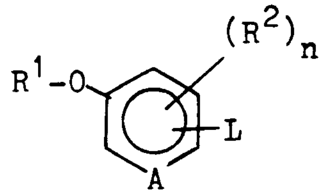

worin L eine Abgangsgruppe wie z.B. Halogen, ($C_1$-$C_4$)-Alkylsulfonyl, Phenylsulfonyl, ($C_1$-$C_4$)-Alkoxycarbonylmethylsulfonyl, ($C_1$-$C_4$)-Alkylsulfinyl, ($C_1$-$C_4$)-Alkylthio sowie den Mesyl- oder Tosylrest bedeutet, mit Verbindungen der Formel

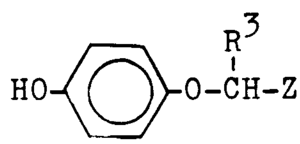

III

oder

b) Verbindungen der Formel

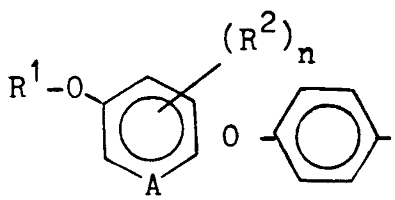

IV

mit Verbindungen der Formel

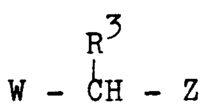

V

worin W für Halogen (vorzugsweise Chlor oder Brom) oder den Mesyl- bzw. Tosylrest steht, umsetzt, oder

c) Verbindungen der Formel I, in der z die -$COOR^4$-Gruppe darstellt, hydriert und die erhaltenen Alkohole (Z = -$CH_2OH$) gewünschtenfalls durch Umsetzung mit Carbonsäuren, Carbonsäurehalogeniden oder Anhydriden in die entsprechenden Carbonsäureester (Z = -$CH_2$-O-C-(O)-$R^{11}$), durch Umsetzung mit Sulfonsäurehalogeniden in Sulfonsäureester (Z = -$CH_2$-O-$SO_2$-$R^{12}$) bzw. durch Umsetzung mit Carbamidsäurehalogeniden oder Isocyanaten in Carbamidsäureester

$$(Z= -CH_2-O-CO-N\begin{matrix} R^6 \\ R^7 \end{matrix})$$

überführt, oder

d) die nach Verfahren a) und b) erhaltenen Verbindungen durch Verseifung, Salzbildung, Säurechloridherstellung und Umsetzung mit Alkoholen, Thiolen und Aminen, Veresterung, Umesterung, Amidierung, Wasserabspaltung, oder -anlagerung oder Schwefelwasserstoffanlagerung in andere erfindungsgemäße Verbindungen der Formel I überführt.

**2.** Herbizide und wachstumsregulierende Mittel, gekennzeichnet durch einen Gehalt an einer Verbindung der Formel I gemäß Anspruch 1.

**3.** Verwendung von Verbindungen der Formel I gemäß Anspruch 1 als Herbizide bzw. als Wachstumsregulatoren.

**4.** Verfahren zur Bekämpfung unerwünschten Pflanzenwuchses, bzw. zur Wachstumsregulierung, dadurch gekennzeichnet, daß man auf die zu behandelnden Pflanzen bzw. Anbauflächen eine wirksame Menge einer Verbindung der Formel I gemäß Anspruch 1 aufbringt.

**Claims**
**Claims for the following Contracting States : BE, CH, DE, FR, GB, GR, IT, LI, NL**

**1.** A compound of the formula I

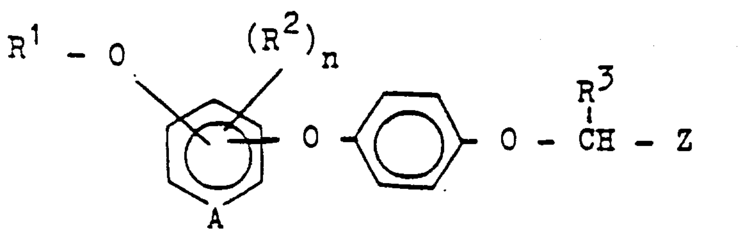

I

in which

| | |
|---|---|
| $R^1$ | denotes halo($C_1$-$C_8$)alkyl, |
| $R^2$ | denotes halogen, |
| n | denotes 0 to 3, |
| A | denotes N, |
| $R^3$ | denotes $CH_3$, |
| Z | denotes a group of the formula |

**$-CO-O-R^4$, $-CO-S-R^5$, $-CO-NR^6R^7$, $-CO-NR^8-NR^9R^{10}$, $-CS-NH_2$, CN**

or

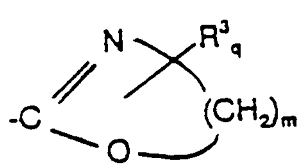

,

| | |
|---|---|
| q | denotes 0 to 3, |
| m | denotes 2 or 3, |
| $R^4$ | denotes H, ($C_1$-$C_{12}$)-alkyl which may optionally be substituted by 1 - 6 halogens, preferably F, Cl or Br, and/or by OH, CN, SCN, ($C_1$-$C_6$)-alkoxy, ($C_1$-$C_4$)-alkylthio, ($C_1$-$C_4$)-alkylsulfinyl, ($C_1$-$C_4$)-alkylsulfonyl, ($C_1$-$C_6$)-alkoxy-($C_2$-$C_6$)-alkoxy, halo-($C_1$-$C_2$)-alkoxy, methoxyethoxyethoxy, ($C_1$-$C_4$)-alkylamino, di-($C_1$-$C_4$)-alkylamino, phenyl, oxiranyl, ($C_1$-$C_4$)-alkoxycarbonyl and/or the radical |

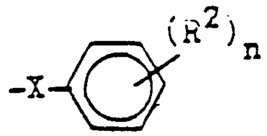 ;

($C_5$-$C_6$)-cycloalkyl which is optionally substituted by halogen or methyl;
($C_3$-$C_6$) alkenyl, halo-($C_3$-$C_6$)-alkenyl or ($C_5$-$C_6$)-cycloalkenyl;
($C_3$-$C_4$)-alkynyl which is optionally mono- or disubstituted by ($C_1$-$C_6$)-alkyl, phenyl, halogen or ($C_1$-$C_2$)-alkoxy;
phenyl which is optionally mono- to trisubstituted by ($C_1$-$C_4$)-alkyl, ($C_1$-$C_4$)-alkoxy, halogen, $NO_2$ or $CF_3$;
furfuryl, tetrahydrofurfuryl, a cation equivalent of an organic or inorganic base, or one of the groups

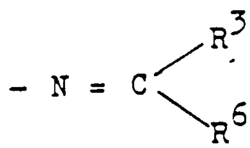

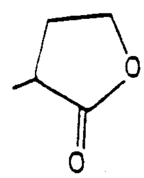

$R^5$ denotes H, ($C_1$-$C_6$)-alkyl, phenyl-($C_1$-$C_2$)-alkyl where the phenyl radical may be mono- or disubstituted by ($C_1$-$C_4$)-alkyl and/or halogen; ($C_3$-$C_6$)-alkenyl, or phenyl which may also be mono- or polysubstituted by ($C_1$-$C_4$)-alkyl and/or halogen,

$R^6$ denotes H, ($C_1$-$C_6$)-alkyl, hydroxy-($C_1$-$C_6$)-alkyl, ($C_5$-$C_6$)-cycloalkyl, or phenyl which is optionally mono- to trisubstituted by ($C_1$-$C_4$)-alkyl, ($C_1$-$C_4$)-alkoxy, halogen or $CF_3$,

$R^7$ has one of the meanings of $R^6$ or may also denote (when $R^6$ = H or ($C_1$-$C_6$)-alkyl) ($C_1$-$C_4$)-alkoxycarbonyl-($C_1$-$C_4$)-alkyl,

$R^6$ and $R^7$ together denote an alkylene chain having 2, 4 or 5 members, in which a $CH_2$ group may be replaced by O, NH or N($CH_3$),

$R^8$ denotes H or $CH_3$,

$R^9$ denotes H, $CH_3$ or $C_2H_5$,

$R^{10}$ denotes H, $CH_3$, $C_2H_5$ or phenyl,
and

X denotes O, S, N-($C_1$-$C_4$)-alkyl, NH, -$OCH_2$-, -$NHCH_2$, -N-(($C_1$-$C_4$)-alkyl)-$CH_2$- or -$SCH_2$-.

**2.** A compound of the formula I as claimed in claim 1, wherein $R^1$ is ($C_1$-$C_2$) haloalkyl and $R^2$ is halogen, preferably chlorine.

**3.** A compound of the formula I as claimed in claim 1 or 2, wherein Z is a radical of the formula -$COOR^4$, -$COSR^5$ or -$CONR^6R^7$, but particularly -$COOR^4$.

**4.** A compound of the formula I as claimed in one of claims 1, 2 or 3, wherein $R^4$ is hydrogen, ($C_1$-$C_6$)-haloalkyl or a cation.

**5.** A process for the preparation of the compounds of the formula I, wherein

a) compounds of the formula

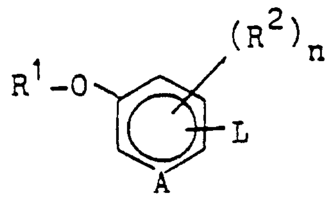

in which L denotes a leaving group such as, for example, halogen, ($C_1$-$C_4$)-alkylsulfonyl, phenylsulfonyl, ($C_1$-$C_4$)-alkoxycarbonylmethylsulfonyl, ($C_1$-$C_4$)-alkylsulfinyl, ($C_1$-$C_4$)-alkylthio and the mesyl or tosyl radical, are reacted with compounds of the formula

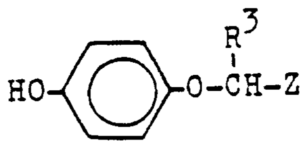

III

or
b) compounds of the formula

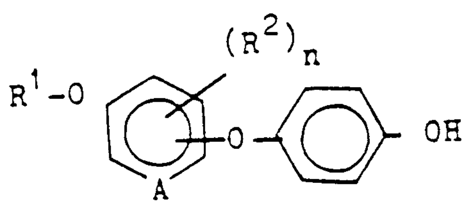

IV

are reacted with compounds of the formula

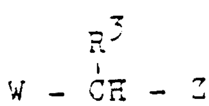

V

in which W represents halogen (preferably chlorine or bromine) or the mesyl or tosyl radical, or
c) compounds of the formula I, in which Z represents the -COOR$^4$ group, are hydrogenated, and the alcohols (Z = $-CH_2OH$) obtained are converted, if desired, into the corresponding carboxylates (Z = $-CH_2-O-C-(O)-R^{11}$) by reaction with carboxylic acids, carboxylic acid halides or anhydrides, into sulfonates (Z = $-CH_2-O-SO_2-R^{12}$) by reaction with sulfonyl halides or into carbamates

(Z= 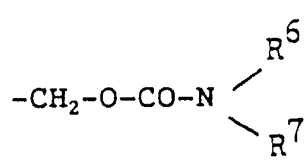 )

by reaction with carbamoyl halides or isocyanates, or

d) the compounds obtained by processes a) and b) are converted into other compounds of the formula I according to the invention by saponification, salt formation, acid chloride preparation and reaction with alcohols, thiols and amines, esterification, transesterification, amidation, elimination of water or hydration, or addition of hydrogen sulfide.

**6.** A herbicidal and growth-regulating agent which contains a compound of the formula I as claimed in claim 1.

**7.** The use of compounds of the formula I as claimed in claim 1 as herbicides or as growth regulators.

**8.** A process for combating undesired plant growth or for growth regulation, wherein an effective amount of a compound of the formula I as claimed in claim 1 is applied to the plants or cultivated areas to be treated.

**Claims for the following Contracting States: AT, ES**

**1.** A process for the preparation of compounds of the formula I

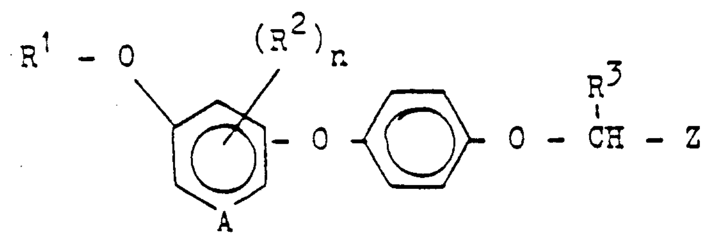 I

in which

| | |
|---|---|
| $R^1$ | denotes halo($C_1$-$C_8$)alkyl, |
| $R^2$ | denotes halogen, |
| n | denotes 0 to 3, |
| A | denotes N, |
| $R^3$ | denotes $CH_3$, |
| Z | denotes a group of the formula |

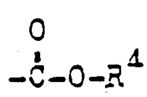, 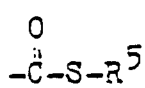, 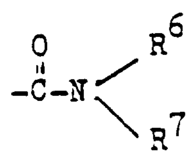, 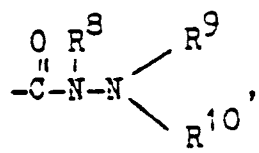

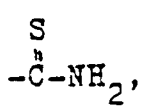, -CN

or

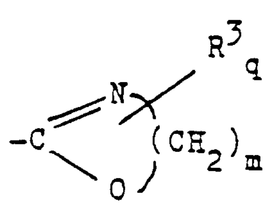 ,

q denotes 0 to 3, m denotes 2 or 3,

$R^4$ denotes H, ($C_1$-$C_{12}$)-alkyl which may optionally be substituted by 1 - 6 halogens, preferably F, Cl or Br, and/or by OH, CN, SCN, ($C_1$-$C_6$)-alkoxy, ($C_1$-$C_4$)-alkylthio, ($C_1$-$C_4$)-alkylsulfinyl, ($C_1$-$C_4$)-alkylsulfonyl, ($C_1$-$C_6$)-alkoxy-($C_2$-$C_6$)-alkoxy, halo-($C_1$-$C_2$)-alkoxy, methoxyethoxyethoxy, ($C_1$-$C_4$)-alkylamino, di-($C_1$-$C_4$)-alkylamino, phenyl, oxiranyl, ($C_1$-$C_4$)-alkoxycarbonyl and/or the radical

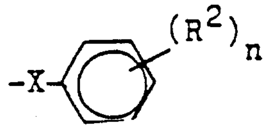 ;

($C_5$-$C_6$)-cycloalkyl which is optionally substituted by halogen or methyl;
($C_3$-$C_6$)-alkenyl, halo-($C_3$-$C_6$)-alkenyl or ($C_5$-$C_6$)-cycloalkenyl;
($C_3$-$C_4$)-alkynyl which is optionally mono- or disubstituted by ($C_1$-$C_6$)-alkyl, phenyl, halogen or ($C_1$-$C_2$)-alkoxy;
phenyl which is optionally mono- to trisubstituted by ($C_1$-$C_4$)-alkyl, ($C_1$-$C_4$)-alkoxy, halogen, $NO_2$ or $CF_3$;
furfuryl, tetrahydrofurfuryl, a cation equivalent of an organic or inorganic base, or one of the groups

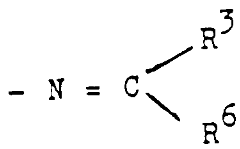 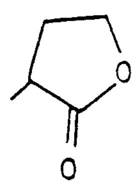 ,

$R^5$ denotes H, ($C_1$-$C_6$)-alkyl, phenyl-($C_1$-$C_2$)-alkyl where the phenyl radical may be mono- or disubstituted by ($C_1$-$C_4$)-alkyl and/or halogen; ($C_3$-$C_6$)-alkenyl, or phenyl which may also be mono- or polysubstituted by ($C_1$-$C_4$)-alkyl and/or halogen,

$R^6$ denotes H, ($C_1$-$C_6$)-alkyl, hydroxy-($C_1$-$C_6$)-alkyl, ($C_5$-$C_6$)-cycloalkyl, or phenyl which is optionally mono- to trisubstituted by ($C_1$-$C_4$)-alkyl, ($C_1$-$C_4$)-alkoxy, halogen or $CF_3$,

$R^7$ has one of the meanings of $R^6$ or may also denote (when $R^6$ = H or ($C_1$-$C_6$)-alkyl ($C_1$-$C_4$)-alkoxycarbonyl-($C_1$-$C_4$)-alkyl,

$R^6$ and $R^7$ together denote an alkylene chain having 2, 4 or 5 members, in which a $CH_2$ group may be replaced by O, NH or N($CH_3$),

$R^8$ denotes H or $CH_3$,

$R^9$ denotes H, $CH_3$ or $C_2H_5$,

$R^{10}$ denotes H, $CH_3$, $C_2H_5$ or phenyl,
and

X denotes O, S, N-($C_1$-$C_4$)-alkyl, NH, -$OCH_2$-, -$NHCH_2$, -N-(($C_1$-$C_4$)-alkyl)-$CH_2$- or -$SCH_2$-, wherein

a) compounds of the formula

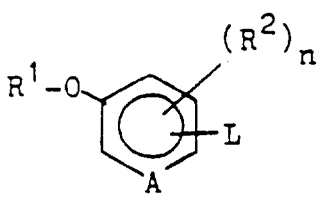

in which L denotes a leaving group such as, for example, halogen, ($C_1$-$C_4$)-alkylsulfonyl, phenylsulfonyl, ($C_1$-$C_4$)-alkoxycarbonylmethylsulfonyl, ($C_1$-$C_4$)-alkylsulfinyl, ($C_1$-$C_4$)-alkylthio and the mesyl or tosyl radical, are reacted with compounds of the formula

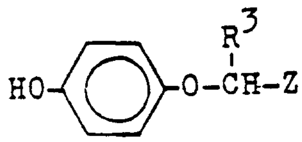

III

or

b) compounds of the formula

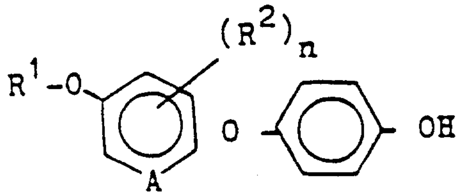

IV

are reacted with compounds of the formula

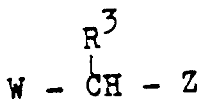

V

in which W represents halogen (preferably chlorine or bromine) or the mesyl or tosyl radical, or

c) compounds of the formula I, in which Z represents the $-COOR^4$ group, are hydrogenated, and the alcohols ($Z = -CH_2OH$) obtained are converted, if desired, into the corresponding carboxylates ($Z = -CH_2O-C-(O)-R^{11}$) by reaction with carboxylic acids, carboxylic acid halides or anhydrides, into sulfonates ($Z = -CH_2-O-SO_2-R^{12}$) by reaction with sulfonyl halides, or into carbamates

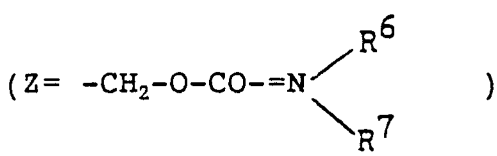

by reaction with carbamoyl halides or isocyanates, or

d) the compounds obtained by processes a) and b) are converted into other compounds of the formula I according to the invention by saponification, salt formation, acid chloride preparation and reaction with alcohols, thiols and amines, esterification, transesterification, amidation, elimination of water or hydration, or addition of hydrogen sulfide.

**2.** A herbicidal and growth-regulating agent which contains a compound of the formula I as claimed in claim 1.

**3.** The use of compounds of the formula I as claimed in claim 1 as herbicides or as growth regulators.

**4.** A process for combating undesired plant growth or for growth regulation, wherein an effective amount of a compound of the formula I as claimed in claim 1 is applied to the plants or cultivated areas to be treated.

**Revendications**

**Revendications pour les Etats contractants suivants : BE, CH, DE, FR, GB, GR, IT, LI, NL**

**1.** Composé de formule I

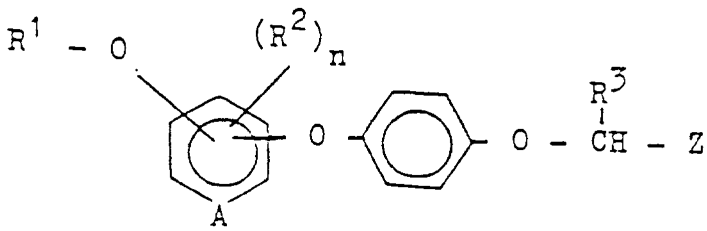

I

dans laquelle

$R^1$ est un radical halogènalkyle en $C_1$-$C_8$,

$R^2$ est un halogène,

n vaut de 0 à 3,

A est N,

$R^3$ est $CH_3$,

Z est un groupe de formule -CO-O-$R^4$, -CO-S-$R^5$, -CO-N$R^6R^7$, -CO-N$R^8$-N$R^9R^{10}$, -CS-$NH_2$, CN ou

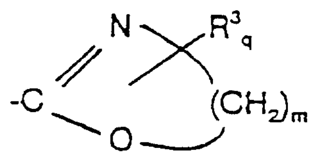

,

q vaut de 0 à 3,

m vaut 2 ou 3,

$R^4$ est H ou un radical alkyle en $C_1$-$C_{12}$ pouvant être éventuellement substitué par 1-6 substituants halogéno, de préférence F, Cl, Br, et/ou OH, CN, SCN, alcoxy en $C_1$-$C_6$,alkylthio en $C_1$-$C_4$, alkylsulfinyle en $C_1$-$C_4$, alkylsulfonyle en $C_1$-$C_4$, (alcoxy en $C_1$-$C_6$)-(alcoxy en $C_2$-$C_6$), halogènalcoxy en $C_1$-$C_2$, méthoxyéthoxyéthoxy, alkylamino en $C_1$-$C_4$, di-(alkyle en $C_1$-$C_4$)-amino, phényle, oxirannyle, (alcoxy en $C_1$-$C_4$)carbonyle et/ou par le radical

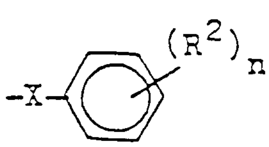

cycloalkyle en $C_5$-$C_6$ éventuellement substitué par des radicaux halogéno ou méthyle ;

alcényle en $C_3$-$C_6$, halogènalcényle en $C_3$-$C_6$ ou cycloalcényle en $C_5$-$C_6$ ;

alcynyle en $C_3$-$C_4$, éventuellement substitué une ou deux fois par des radicaux alkyle en $C_1$-$C_6$, phényle, halogéno ou alcoxy en $C_1$-$C_2$ ;

phényle éventuellement substitué par un à trois radicaux alkyle en $C_1$-$C_4$, alcoxy en $C_1$-$C_4$, halogéno, $NO_2$ ou $CF_3$;

furfuryle, tétrahydrofurfuryle, un équivalent cationique d'une base organique ou minérale, ou l'un des groupes

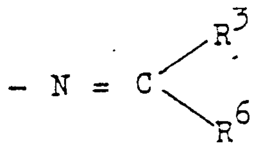

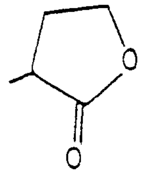

,

$R^5$ est H ou un radical alkyle en $C_1$-$C_6$, phényl-(alkyle en $C_1$-$C_2$), le radical phényle pouvant être une ou deux fois substitué par des radicaux alkyle en $C_1$-$C_4$ et/ou halogéno ; alcényle en $C_3$-$C_6$ ou phényle pouvant aussi être une ou plusieurs fois substitué par des radicaux alkyle en $C_1$-$C_4$ et/ou halogéno,

$R^6$ est H ou un radical alkyle en $C_1$-$C_6$, hydroxyalkyle en $C_1$-$C_6$, cycloalkyle en $C_5$-$C_6$ ou phényle, éventuellement substitué par un à trois substituants alkyle en $C_1$-$C_4$, alcoxy en $C_1$-$C_4$, halogéno ou $CF_3$,

$R^7$ a l'une des significations de $R^6$ ou encore, quand $R^6$ est H ou un radical alkyle en $C_1$-$C_6$, peut aussi représenter un radical (alcoxy en $C_1$-$C_4$)-carbonyl-(alkyle en $C_1$-$C_4$),

$R^6$ et $R^7$ forment ensemble un enchaînement alkylène ayant 2, 4 ou 5 chaînons, dans lequel un groupe $CH_2$ peut être remplacé par O, NH ou $N(CH_3)$,

$R^8$ est H ou $CH_3$,

$R^9$ est H, $CH_3$ ou $C_2H_5$,

$R^{10}$ est H, $CH_3$, $C_2H_5$ ou le radical phényle,

et encore

X est O, S ou un radical N-(alkyle en $C_1$-$C_4$), NH, $-COH_2-$, $-NHCH_2$, -N-(alkyle en $C_1$-$C_4$)-$CH_2$- ou $-SCH_2-$.

2. Composés de formule I selon la revendication 1, caractérisés en ce que $R^1$ est un radical halogènalkyle en $C_1$-$C_2$ et $R^2$ est un halogène, de préférence le chlore.

3. Composés de formule I selon les revendications 1 ou 2, caractérisés en ce que Z est un radical de formule $-COOR^4$, $-COSR^5$ ou $-CONR^6R^7$, mais en particulier $-COOR^4$.

4. Composés de formule I selon l'une des revendications 1, 2 ou 3, caractérisés en ce que $R^4$ est un hydrogène ou un radical halogènalkyle en $C_1$-$C_6$, ou un cation.

5. Procédé pour préparer les composés de formule I, caractérisé en ce qu'on fait réagir

a) des composés de formule

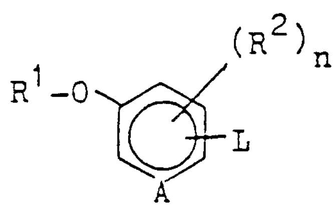

dans laquelle L est un groupe éliminable, tel par exemple qu'un groupe halogéno, alkylsulfonyle en $C_1$-$C_4$, phénylsulfonyle, (alcoxy en $C_1$-$C_4$)-carbonylméthylsulfonyle, alkylsulfinyle en $C_1$-$C_4$, alkylthio en $C_1$-$C_4$, ou encore le radical mésyle ou tosyle, avec des composés de formule

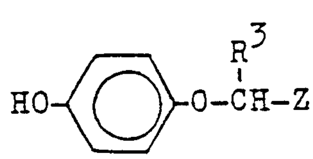

III

ou bien
b) des composés de formule

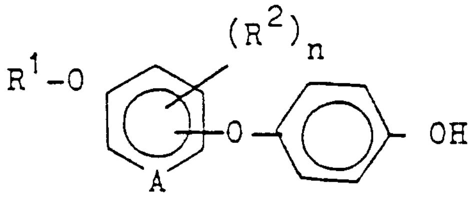

IV

avec des composés de formule

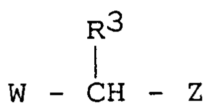

V

dans laquelle W est un halogène (de préférence le chlore ou le brome), ou représente le radical mésyle ou tosyle, ou encore
c) en ce qu'on hydrogène des composés de formule I dans laquelle z représente le groupe $-COOR^4$, et on convertit les alcools obtenus ($Z = -CH_2OH$), éventuellement par réaction avec des acides carboxyliques, des halogénures ou des anhydrides d'acides carboxyliques, en les carboxylates correspondants ($Z = -CH_2\text{-}O\text{-}C\text{-}(O)\text{-}R^{11}$), par réaction avec des halogénures d'acides sulfoniques en sulfonates ($Z = -CH_2\text{-}O\text{-}SO_2\text{-}R^{12}$) ou encore, par réaction avec des halogénures ou des isocyanates d'acides carbamiques, en des carbamates ($Z = -CH_2\text{-}O\text{-}CO\text{-}NR^6R^7$), ou bien
d) en ce qu,on convertit les composés obtenus selon les modes opératoires a) et b), après saponification, salification, production du chlorure d'acide et réaction avec des alcools, des thiols et des amines, estérification, transestérification, amidation, élimination ou fixation de l'eau ou fixation d'hydrogène sulfuré, en d'autres composés de formule I selon l'invention.

**6.** Herbicides et régulateurs de croissance, caractérisés en ce qu'ils contiennent un composé de formule I selon la revendication 1.

**7.** Utilisation de composés de formule I selon la revendication 1 comme herbicides ou régulateurs de croissance.

**8.** Procédé pour lutter contre une croissance indésirable de végétaux ou pour réguler leur croissance, caractérisé en ce qu'on applique sur les végétaux à traiter ou sur les surfaces cultivées une quantité efficace d'un compose de formule I selon la revendication 1.

**Revendications pour les Etats contractants suivants : AT, ES**

**1.** Procédé pour préparer des composés de formule I

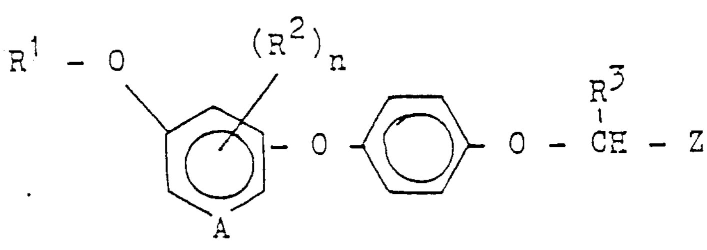

I

dans laquelle
$R^1$ est un radical halogènalkyle en $C_1$-$C_8$,

$R^2$ est un halogène,
n vaut de 0 à 3,
A est N,
$R^3$ est $CH_3$,
Z est un groupe de formule $-CO-O-R^4$, $-CO-S-R^5$, $-CO-NR^6R^7$, $-CO-NR^8-NR^9R^{10}$, $-CS-NH_2$, CN ou

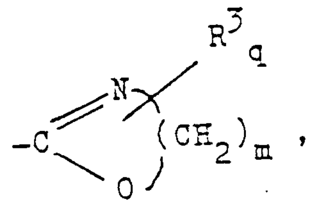

q vaut de 0 à 3,
m vaut 2 ou 3,
$R^4$ est H ou un radical alkyle en $C_1$-$C_{12}$ pouvant être éventuellement substitué par 1-6 substituants halogéno, de préférence F, Cl, Br, et/ou OH, CN, SCN, alcoxy en $C_1$-$C_6$,alkylthio en $C_1$-$C_4$, alkylsulfinyle en $C_1$-$C_4$, alkylsulfonyle en $C_1$-$C_4$, (alcoxy en $C_1$-$C_6$)-(alcoxy en $C_2$-$C_6$), halogènalcoxy en $C_1$-$C_2$, méthoxyéthoxyéthoxy, alkylamino en $C_1$-$C_4$, di-(alkyle en $C_1$-$C_4$)-amino, phényle, oxirannyle, (alcoxy en $C_1$-$C_4$)carbonyle et/ou par le radical

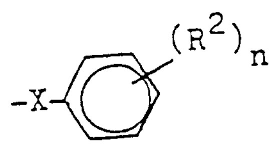

cycloalkyle en $C_5$-$C_6$ éventuellement substitué par des radicaux halogéno ou méthyle ;
alcényle en $C_3$-$C_6$, halogènalcényle en $C_3$-$C_6$ ou cycloalcényle en $C_5$-$C_6$ ;
alcynyle en $C_3$-$C_4$, éventuellement substitué une ou deux fois par des radicaux alkyle en $C_1$-$C_6$, phényle, halogéno ou alcoxy en $C_1$-$C_2$ ;
phényle éventuellement substitué par un à trois radicaux alkyle en $C_1$-$C_4$, alcoxy en $C_1$-$C_4$, halogéno, $NO_2$ ou $CF_3$;
furfuryle, tétrahydrofurfuryle, un équivalent cationique d'une base organique ou minérale, ou l'un des groupes

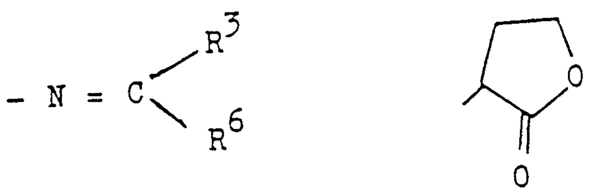

,

$R^5$ est H ou un radical alkyle en $C_1$-$C_6$, phényl-(alkyle en $C_1$-$C_2$), le radical phényle pouvant être une ou deux fois substitué par des radicaux alkyle en $C_1$-$C_4$ et/ou halogéno ; alcényle en $C_3$-$C_6$ ou phényle pouvant aussi être une ou plusieurs fois substitué par des radicaux alkyle en $C_1$-$C_4$ et/ou halogéno,
$R^6$ est H ou un radical alkyle en $C_1$-$C_6$, hydroxyalkyle en $C_1$-$C_6$, cycloalkyle en $C_5$-$C_6$ ou phényle, éventuellement substitué par un à trois substituants alkyle en $C_1$-$C_4$, alcoxy en $C_1$-$C_4$, halogéno ou $CF_3$,
$R^7$ a l'une des significations de $R^6$ ou encore, quand $R^6$ est H ou un radical alkyle en $C_1$-$C_6$, peut aussi représenter un radical (alcoxy en $C_1$-$C_4$)-carbonyl-(alkyle en $C_1$-$C_4$),
$R^6$ et $R^7$ forment ensemble un enchaînement alkylène ayant 2, 4 ou 5 chaînons, dans lequel un groupe $CH_2$ peut être remplacé par O, NH ou $N(CH_3)$,
$R^8$ est H ou $CH_3$,

$R^9$ est H, $CH_3$ ou $C_2H_5$,
$R^{10}$ est H, $CH_3$, $C_2H_5$ ou le radical phényle,
et encore
X est O, S ou un radical N-(alkyle en $C_1$-$C_4$), NH, -$COH_2$-, -$NHCH_2$, -N-(alkyle en $C_1$-$C_4$)-$CH_2$- ou -$SCH_2$-, caractérisé en ce qu on fait réagir
a) des composés de formule

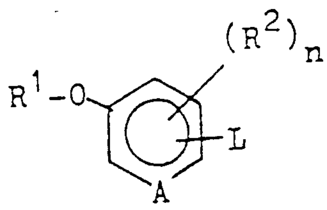

dans laquelle L est un groupe éliminable, tel par exemple qu'un groupe halogéno, alkylsulfonyle en $C_1$-$C_4$, phénylsulfonyle, (alcoxy en $C_1$-$C_4$)-carbonylméthylsulfonyle, alkylsulfinyle en $C_1$-$C_4$, alkylthio en $C_1$-$C_4$, ou encore le radical mésyle ou tosyle, avec des composés de formule

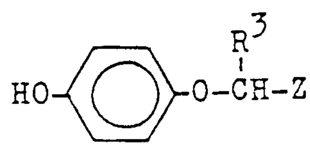

III

ou bien
b) des composés de formule

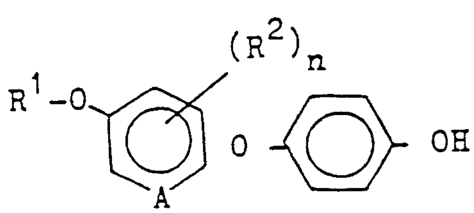

IV

avec des composés de formule

$$W - \overset{R^3}{\overset{|}{C}H} - Z \qquad V$$

dans laquelle W est un halogène (de préférence le chlore ou le brome), ou représente le radical mésyle ou tosyle, ou encore
c) en ce qu'on hydrogène des composés de formule I dans laquelle z représente le groupe -$COOR^4$, et on convertit les alcools obtenus (Z = -$CH_2OH$), éventuellement par réaction avec des acides carboxyliques, des halogénures ou des anhydrides d'acides carboxyliques, en les carboxylates correspondants (Z = -$CH_2$-O-C-(O)-$R^{11}$), par réaction avec des halogénures d'acides sulfoniques en sulfonates (Z = -$CH_2$-O-$SO_2$-$R^{12}$) ou encore, par réaction avec des halogénures ou des isocyanates d'acides carbamiques, en des carbamates (Z = -$CH_2$-O-CO-$NR^6R^7$), ou bien
d) en ce qu on convertit les composés obtenus selon les modes opératoires a) et b), après saponification, salification, production du chlorure d'acide et réaction avec des alcools, des thiols et des amines, estérification, transestérification, amidation, élimination ou fixation de l'eau ou fixation d'hydrogène sulfuré, en d'autres composés de formule I selon l'invention.

**2.** Herbicides et régulateurs de croissance, caractérisés en ce qu'ils contiennent un composé de formule I selon la revendication 1.

**3.** Utilisation de composés de formule I selon la revendication 1 comme herbicides ou régulateurs de croissance.

**4.** Procédé pour lutter contre une croissance indésirable de végétaux ou pour réguler leur croissance, caractérisé en ce qu'on applique sur les végétaux à traiter ou sur les surfaces cultivées une quantité efficace d'un composé de formule I selon la revendication 1.